# EUROPÄISCHE PATENTSCHRIFT

(11) **EP 2 996 586 B1**
(45) Veröffentlichungstag und Bekanntmachung des Hinweises auf die Patenterteilung: **12.07.2017**
(21) Anmeldenummer: 14728468.1
(22) Anmeldetag: 14.05.2014
(51) Int. Cl.: A61B 17/32, A61B 17/00

(54) **KONNEKTIERUNG FÜR EINEN MORCELLATOR**
CONNECTION FOR A MORCELLATOR
CONNEXION POUR UN MORCELLATEUR

(30) Priorität: 17.05.2013 DE 202013102186 U; 16.08.2013 DE 202013007298 U
(43) Veröffentlichungstag der Anmeldung: 23.03.2016
(73) Patentinhaber: Trokamed GmbH, 78187 Geisingen (DE)
(72) Erfinder: TRÖNDLE, Karlheinz, 78187 Geisingen (DE)
(74) Vertreter: Patentanwälte und Rechtsanwalt Weiß, Arat & Partner mbB
(86) Internationale Anmeldenummer: PCT/EP2014/059863
(87) Internationale Veröffentlichungsnummer: WO 2014/184251

(56) Entgegenhaltungen:
- DE-A1-102008 036 420
- DE-A1-102010 037 974
- US-A- 4 461 305
- US-A1- 2005 010 238
- US-A1- 2008 039 884
- US-A1- 2011 251 597

## Beschreibung

Die Erfindung betrifft eine Konnektierung für einen Morcellator nach dem Oberbegriff des Schutzanspruchs 1.

### Stand der Technik

Aus dem Stand der Technik sind verschiedene Konnektierungen für Morcellatoren bekannt und gebräuchlich. Die Erfindung ist überall dort anzuwenden, wo über ein sich drehendes Schneidrohr, welches insbesondere als Schneidrohr ausgestaltet ist, eine Tätigkeit im Körper eines Lebewesens durchgeführt wird. Heute werden derartige medizinische Instrument vor allem bei endoskopischen Eingriffen verwendet. Sie dienen dazu, grössere Gewebeanteile zu entfernen.

In diesem Zusammenhang wird auf die DE 10 2008 036 420 A1 hingewiesen, welche eine Vorrichtung zum Ausschneiden und Entfernen von Gewebezylindern aus einem Gewebe beschreibt.

Weiter wird auf die US 2011/251597 A1 hingewiesen, welche einen Bohrer zeigt, welcher innerhalb einer Hülse drehbar gelagert ist, womit eine Betätigung des Bohrers ohne Beteiligung der Hülse möglich ist.

Daneben wird auf die US 4 461 305 A1 hingewiesen, welche zur Entnahme von Gewebe ein Handstück mit einer austauschbaren Hülse aufzeigt, wobei in und auf der Hülse unterschiedliche Werkzeuge anbringbar und betätigbar sind.

Ausserdem wird der Vollständigkeit auf die US 2008/039884 A1 und die US 2005/010238 A1 hingewiesen, welche ähnliche Vorrichtungen aufzeigen.

Speziell der Morcellator weist als Schneidrohr ein Schneidrohr auf, welches am distalen Ende eine Schneide besitzt. Dieses Schneidrohr wird beispielsweise durch ein Endoskop in den Körper eingeführt und dann in Drehbewegung versetzt. Hierdurch entfernt die Schneide Gewebeanteile, die dann durch das Schneidrohr selbst aus dem Körper entnommen werden. Hierzu kann das Gewebeteil abgesaugt oder aber auch durch ein weiteres medizinisches Instrument entfernt werden, welches dann beispielsweise durch ein Ventilmodul hindurch in das Schneidrohr und durch dieses hindurch geführt wird.

Ein Morcellator nach dem Stand der Technik ist insbesondere in der DE 10 2010 037 974 A1 beschrieben. Die vorliegende Erfindung soll eine Fortbildung des dort gezeigten Morcellators sein.

### Aufgabe der Erfindung

Aufgabe der Erfindung ist es eine Konnektierung für einen Morcellator nach der DE 10 2010 037 974 A1 zur Verfügung zu stellen, welcher die Handhabung durch den Nutzer weiter vereinfacht und somit zur Erhöhung der Sicherheit während der Nutzung beizutragen.

### Lösung der Aufgabe

Zur Lösung der Aufgabe führen die Merkmale nach dem Anspruch 1.

Zunächst wird bzgl. der Einzelheiten auf die Offenbarung der DE 10 2010 037 974 A1 verwiesen. Dort ist ein Morcellator beschrieben.

Ein erfindungsgemässes Konnektionselement dient zur Verbindung mit einem Morcellator. Der Morcellator weist hierbei ein Handmodul, ein Schneidrohr und eine Hülse auf. Das Handmodul wiederum weist ein Konnektionsstück auf. Die Hülse weist ein Konnektionsring auf. Der Konnektionsring ist auf das Konnektionsstück aufclipsbar. Das bedeutet im Einzelnen, dass die Hülse mit dem Handmodul durch Aufclipsen verbunden werden kann. Dies geschieht bevorzugt durch eine Wippraste, welche manuell betätigt werden kann. Durch die Wippraste wird der Innenumfang des Konnektionsrings vergrössert. Nachdem der Konnektionsring auf das Konnektionsstück aufgesetzt wurde, wird die Wippraste losgelassen und greift in eine Nut des Konnektionsstücks. Dadurch wird eine Verbindung des Konnektionsrings mit dem Konnektionsstück erreicht. Erfindungsgemäß umfasst das Konnektionsstück einen Anflanschring mit einer Ausnehmung. Der Anflanschring weist erfindungsgemäß mehrere Ausnehmungen auf. Sie sind entweder über einen Teil des Umfangs des Anflanschrings verteilt, oder über den gesamten Umfang des Anschlagsrings verteilt. Die Anzahl der Ausnehmungen auf dem Anflanschring ist beliebig. Ein Flanschnocken kann mit den Ausnehmungen des Konnektionsstücks in Eingriff gebracht werden.

Das führt dazu, dass durch die Auswahl einer bestimmten Ausnehmung und dem Einbringen des Flanschnockens in die Ausnehmung, der Konnektionsring an einer bestimmten Stelle des Umfangs des Konnektionsstücks fixiert wird. Der Flanschnocken bildet weiter im wesentlichen eine Linie mit einer Abdeckung der Hülse. Die Abdeckung stellt eine Verlängerung des Umfangs der Hülse an einem Teil dar. Die Abdeckung wird benötigt, um bei einem rotierenden Schneidrohr keine Rotationsbewegungen an das bearbeitete Gewebe weiterzugeben. Das bearbeitete Gewebe kann sich hierdurch nicht an einer Schneide festlegen und mitrotieren, da die Abdeckung über einen bestimmten Bereich einer Schneide übergreift.

Ein erfindungsgemässes Konnektionselement weist weiter eine Positionsanzeige in Form einer Wulst auf. Die Wulst zeigt hierbei die Position des Flanschnockens und somit der Abdeckung an. Wird bspw. auf den Flanschring eine Ausnehmung auf 12 Uhr ausgewählt, so weist der Nutzer auch bei Gebrauch, wenn also die Abdeckung im Inneren des menschlichen Körper ist, wo sich die Abdeckung befindet. Sollte dann eine Entkoppelung des Konnektionsrings und des Konnektionsstücks erfolgen, um eine Umsetzung des Flanschnockens in eine andere Ausnehmung des Flanschrings zu vollziehen, hätte der Nutzer unter Umständen eine komfortablere Handschaltung. Dies wiederum würde zu einer Erleichterung seiner Arbeit führen. Dabei hat er durch die Wulst eine genaue Positionsanzeige der Abdeckung. Weiter ist das Schneidrohr über einen Rastnocken mit dem Handmodul verclipsbar. Dabei greift der Rastnocken in eine entsprechende Ausnehmung innerhalb des Handmoduls ein.

Daneben ist ein Betätigungsknopf an dem Handmodul vorhanden. Der Betätigungsknopf bewirkt eine Schraubbewegung des Konnektionsstücks an dem Handmodul. In dem Zustand, wenn der Konnektionsring auf das Konnektionsstück aufgeclipst ist, wird nicht nur das Konnektionsstück, sondern auch der Konnektionsring mit der dazugehörigen Hülse weg von dem Handmodul versetzt oder hin zu dem Handmodul versetzt. Dies hängt von der jeweils durch den Betätigungsknopf gewünschten Position ab. Durch die Beabstandung des Konnektionsstücks und des damit verbundenen Konnektionsrings von dem Handmodul wird auch erreicht, dass die Hülse über die Schneide geschoben wird. Bei einer reversiblen Betätigung, wird wiederum die Hülse zu dem Handmodul hingezogen und gibt einen Teil der Schneide wieder frei.

Dies bedeutet, dass beispielsweise das Handmodul immer gleich bleiben kann, während das Schneidrohr ausgetauscht wird. Das Schneidrohr kann gegen ein gleichartiges Schneidrohr bei Verschleiss oder Verschmutzung ausgetauscht werden, denkbar ist aber auch der Einsatz unterschiedlich ausgestalteter Schneidrohre für unterschiedliche medizinische Tätigkeiten.

In einem bevorzugten Ausführungsbeispiel ist das Schneidrohr als hohl ausgebildet, welches um seine Achse drehbar mit dem Handmodul verbunden ist.
Bevorzugt soll die lösbare Verbindung zwischen Schneidrohr und Handmodul als Rastverbindung ausgestaltet sein. Das bedeutet, dass das Schneidrohr einfach in das Handmodul eingesetzt ist und einen Rast- oder Klickverschluss eingeht. Ebenso kann das Schneidrohr aus dem Handmodul entfernt werden, indem entweder am Schneidrohr und/oder am Handmodul gezogen wird.
Eine Rastverbindung wird dadurch bewirkt, dass an einem Element eine bevorzugt als Rastfeder ausgebildete Raste angeordnet ist, die mit einem entsprechenden Rastnocken in dem Handmodul zusammenwirkt. Dabei kann die Raste bzw. Rastfeder selbst aus dem Schneidrohr herausgeformt sein und ist bevorzugt selbstfedernd ausgebildet, so dass sie beim Einsetzen des Schneidrohrs in das Handmodul nach innen nachgibt und dann den Rastnocken hinterschnappt. Ebenso gibt sie bei Herausziehen des Schneidrohrs aus dem Handmodul nach innen nach und überfährt den Rastnocken. Der Rastnocken kann dabei ringförmig als Innenring in einem Aufnahmeelement in dem Handmodul vorgesehen sein, jedoch sind auch andere Möglichkeiten denkbar.
Des weiteren soll dem Schneidrohr ein Aussenzahnkranz zugeordnet sein, der mit einem Innenzahnkranz in dem Handmodul zusammenwirkt. Dieser Innenzahnkranz steht über ein Ringkegelrad mit einem Kegelantriebsrad in Verbindung, welches wiederum bevorzugt auf einer Drehachse eines Rotors aufsitzt. Hierdurch wird die Drehbewegung der Drehachse auf das Schneidrohr übertragen.

Zur Abstützung beim Drehen sind dem Schneidrohr bevorzugt zwei beabstandete Gleitlagerringe aufgesetzt, die in entsprechenden Gleitlagern in dem Aufnahmeelement in dem Handmodul drehen.
Für die letzt beschriebene Anordnung wird auch selbstständig Schutz begehrt, da vorgesehen ist, dass das Schneidrohr mit den Gleitringen in den Gleitlagern mit mindestens 0,2 Nm jedoch maximal 5 Nm lagert. Die Gleitringe sind kraftschlüssig auf das Schneidrohr aufgepresst, ferner ist daran gedacht, das Schneidrohr insbesondere in dem proximalen Bereich, der in das Handmodul eingesetzt wird, mehrteilig auszubilden.

Ein weiterer Gedanke der vorliegenden Offenbarung betrifft die Ausgestaltung des distalen Endes des Schneidrohrs. Hier soll ein Abschnitt aus gehärtetem Flachstahl vorgesehen sein, der auch die Schneide ausbildet. Dabei besteht die Möglichkeit, dass dieses aus Flachstahl gebildete Schneidelement das Schneidrohr radial umfasst oder in das Schneidrohr eingesetzt ist. Ferner kann es auch direkt stumpf an das Schneidrohr angesetzt sein.
Auf der dem Schneidrohr gegenüberliegenden Seite des Handmoduls ist bevorzugt eine Ventileinheit eingesetzt. Diese kann eine bajonettartige Verbindung mit dem Handmodul eingehen. In der Ventileinheit befindet sich ein Ventil, welches das Einsetzen eines weiteren medizinischen Instruments zulässt, jedoch dieses Instrument so luftdicht wie möglich umschliesst.
Ein medizinisches Instrument im Rahmen dieser Erfindung ist bevorzugt ein chirurgisches Instrument, insbesondere ein im Rahmen der minimal-invasiven Chirurgie verwendetes Instrument. Beispielhaft aber nicht abschliessend sei hierbei ein Morcellator erwähnt.

### Figurenbeschreibung

Weitere Vorteile, Merkmale und Einzelheiten ergeben sich aus der nachfolgenden Beschreibung eines erfindungsgemässen Ausführungsbeispiels. Die Figuren zeigen im Einzelnen in
Figur 1 einen zerlegten Morcellator;
Figur 2 einen teilweise zerlegten Morcellator nach Figur 1;
Figur 3 einen teilweise zerlegten Morcellator;
Figur 4 einen zusammengebauten Morcellator;
Figur 5 eine andere vergrösserte Ansicht eines Teils der Figur 3;
Figur 6 eine perspektivische Ansicht einer Schutzhülse S mit einem darin befindlichen medizinischen Instrument I in Gebrauchslage,
Figur 7 eine Schutzhülse S und ein medizinisches Instrument I mit einem Schaft 15 nach Figur 6 in Ausgangslage,
Figur 8 eine perspektivische Teilansicht des proximalen Endes der Schutzhülse S nach Figur 6,
Figur 9 eine Ansicht nach Figur 8 in Ausgangslage ohne medizinisches Instrument I,
Figur 10 eine geschnittene Seitenansicht einer Schutzhülse S in Ausgangslage,
Figur 11 eine teilweise geschnittene Seitenansicht eines Ventils 4 in Ausgangslage,
Figur 12 eine perspektivische Ansicht eines Ventils 4 im auseinander gebauten Zustand,
Figur 13 eine perspektivische Ansicht eines Ventils 4 im zusammengebauten Zustand,
Figur 14 eine perspektivische Ansicht einer Schutzhülse S mit Ventil 4 und Abdichtelement 5 in einer ersten Ausgangslage,
Figur 15 eine perspektivische Ansicht einer Schutzhülse S mit Ventil 4 und Abdichtelement 5 in einer zweiten Ausgangslage.

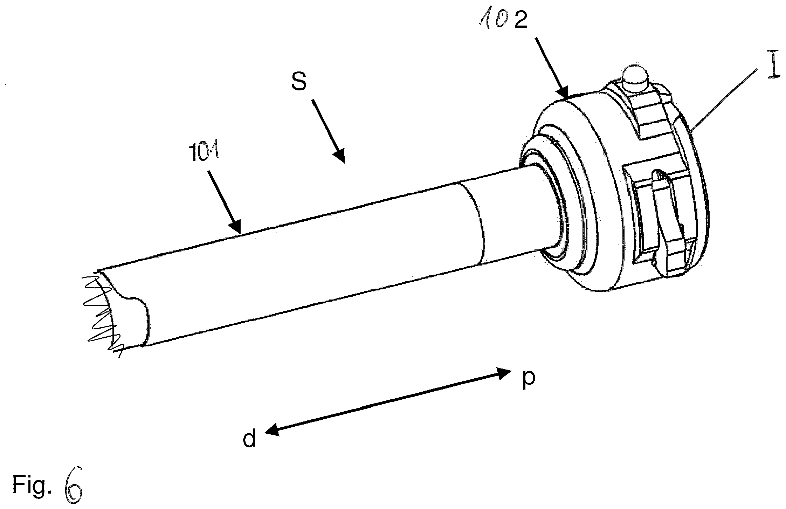

Die Figuren 6 bis 15 bezogen auf eine Schutzhülse sind nicht Teil dieser Erfindungsidee.

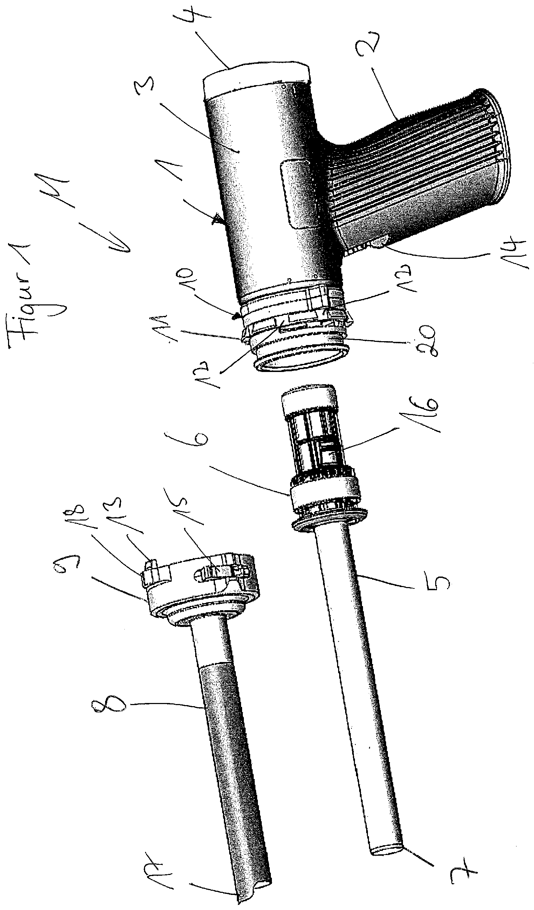

In Figur 1 ist ein erfindungsgemässes Konnektionselement für einen Morcellator M gezeigt. Das Konnektionselement besteht aus einem Konnektionsring 9 und einem Konnektionsstück 10. In diesem Ausführungsbeispiel ist der Konnektionsring 9 Teil einer Hülse 8, wohingegen das Konnektionsstück Teil eines Handmoduls 1 ist. Der Morcellator M besteht hierbei aus dem Handmodul 1, einem Schneidrohr 5 und der Hülse 8. Der Konnektionsring 9 ist auf das Konnektionsstück aufclipsbar ist. Dies bedeutet, dass in der Regel der Innenumfang des Konnektionsrings 9 grösser ist, als der Aussenumfang des Konnektionsstücks 10.
Ausserdem ist ein Anflanschring 11 zu erkennen, welcher als Teil des Konnektionsstücks 10 ausgebildet ist. Der Anflanschring 11 weist verschiedenen Ausnehmungen 12 auf. Diese verschiedenen Ausnehmungen 12 sind hier über den Gesamtumfang des Anflanschrings 11 verteilt. Weiter ist ein Flanschnocken 13 zu erkennen, welcher als Teil des Konnektionsrings 9 ausgebildet ist. In Figur 1 ist weiter gezeigt, dass das Schneidrohr 5 distalseitig eine Schneide 7 aufweist. Daneben ist ein Antriebsanschluss 6 gezeigt, welcher über einen Rastnocken 16 mit dem Handmodul 1 verbunden werden kann. Das Handmodul 1 weist im Bereich des Führungsteils 3 einen

Antrieb und ein Getriebe auf, welches dazu führt, dass die Schneide 7 in Rotation gesetzt werden kann. Weiter ist ein Ventilmodul 4 zu erkennen. Das Ventilmodul 4 ist hierbei andernends des Führungsteils 3 zu dem Konnektionsstück 10 angebracht. Neben dem Führungsteil 3 weist das Handmodul 1 auch ein Griffteil 2 auf. Das Griffteil 2 wiederum umfasst einen Betätigungsknopf 14. Weiter ist in der Figur 1 eine Wippraste 15 gezeigt, welche durch manuelle Betätigung den Innenumfang des Konnektionsrings 9 vergrössert und somit ein Aufsetzen des Konnektionsrings 9 auf das Konnektionsstück 10 erlaubt. Nachdem aufsetzen des Konnektionsrings 9 auf das Konnektionsstück 10, kann die Wippraste 15 wieder manuell gelöst werden. Dabei wird sie dann über einen Federmechanismus in eine Nut 20 des Konnektionsstücks 10 gedrückt und liegt somit den Konnektionsring 9 an dem Konnektionsstück 10 fest.

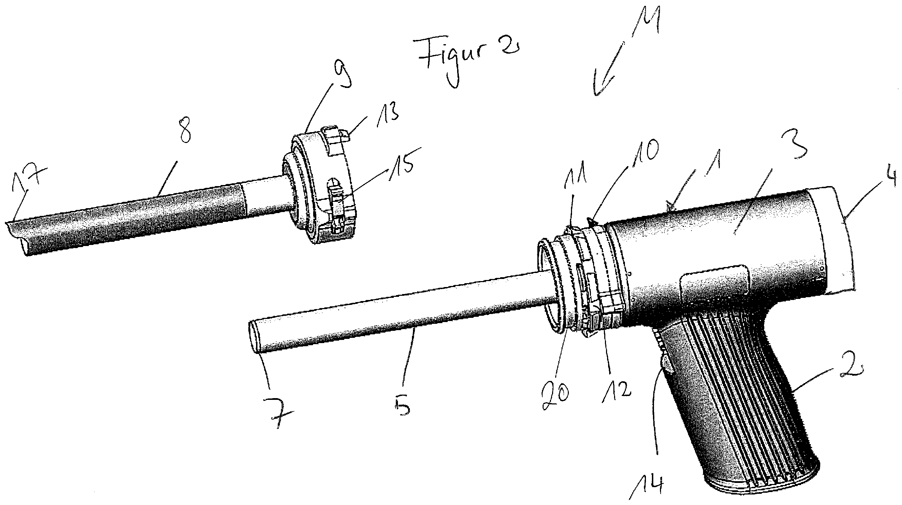

In Figur 2 ist gezeigt, wie das Schneidrohr 5 mit den dazugehörigen Merkmalen in das Führungsteil 3 eingeclipst ist. Weiter ist zu erkennen, wie die Hülse 8 andernends des Konnektionsrings 9 eine Abdeckung 17 aufweist. Die Abdeckung 17 bildet hierbei im wesentlichen eine Linie mit dem Flanschnocken 13. Die Abdeckung 17 ist eine distalseitig teilweise Verlängerung des Umfangs der Hülse 8.

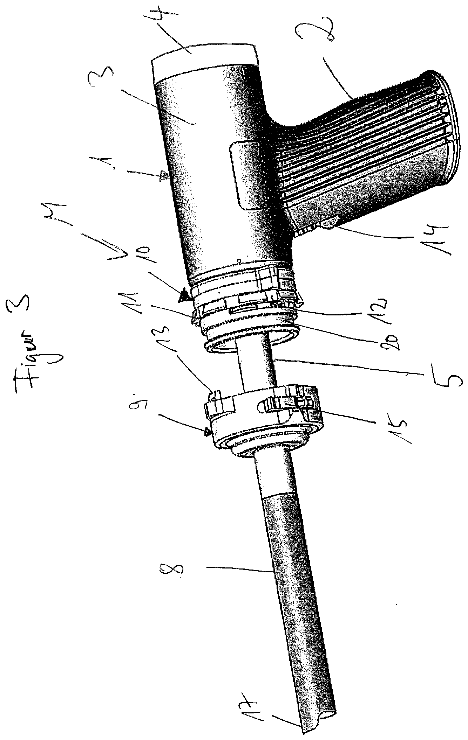

In Figur 3 ist nun gezeigt, wie die Hülse 8 ein teilweise auf das Schneidrohr 5 gezogen wurde.

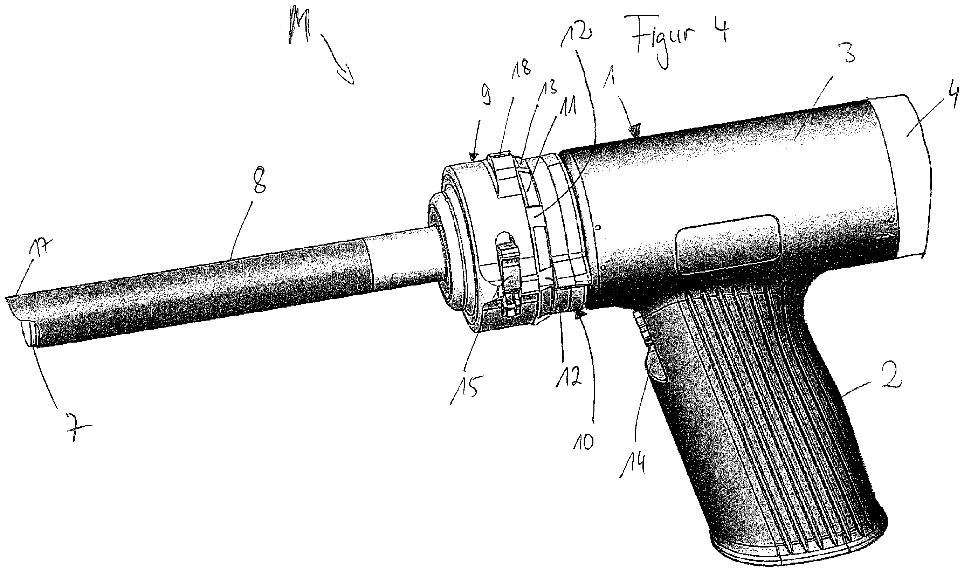

In Figur 4 ist letztendlich gezeigt, wie der Konnektionsring 9 mit dem Konnektionsstück 10 verbunden wurde. Dabei greift der Flanschnocken 13 nun in eine vordefinierte Ausnehmung 12 des Konnektionsstücks 10 ein, wobei die Wulst 18 in diesem Fall immer die genaue Position der Abdeckung 17 zeigt. In Figur 4 ist das Konnektionsstück 10 nicht zu dem Handmodul 1 durch Betätigen des Betätigungsknopfes 14 beabstandet, so dass die Schneide 7 distalseitig teilweise aus der Hülse 8 herausragt, soweit sie von der Abdeckung 17 nicht überdeckt ist.

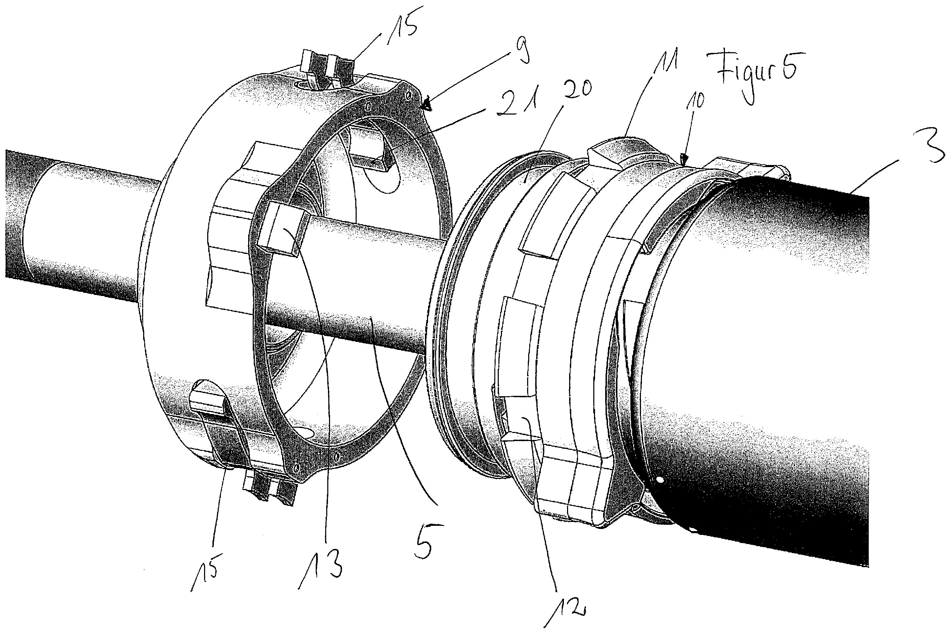

In Figur 5 ist nochmals gezeigt eine vergrösserte Detailansicht der Figur 3 aus einer anderen Perspektive gezeigt. Dort ist bspw. gut zu erkennen, wie die Wippraste 15 einen Einraster 21 aufweist, welche dann in die Nut 20 des Konnektionsstücks 10 eingreift.

In ein offenes Ende des Führungsteils 3 ist das Ventilmodul 4 und in das andere offene Ende das Schneidrohr 5 in Gebrauchslage eingesetzt, wobei das Schneidroh andernends des Antriebsanschlusses 6 die Schneide 7 aufweist. In dem Griffteil 2 befindet sich der Antrieb. Dieser Antrieb wirkt mit einem Ringkegelrad zusammen, das ein Aufnahmeelement in dem Führungsteil des Handmoduls 1 umfängt, wobei ein ringförmig ausgestalteter Rastnocken in eine entsprechende Ringnut in dem Aufnahmeelement eingreift. Diese Ringnut bildet in das Innere des Aufnahmeelements wiederum eine ringförmige Rastnocke aus.

In Gebrauchslage übergreift das Ringkegelrad mit einem Innenzahnkranz einen Aussenzahnkranz, der auf das als Schneidrohr 5 aufgesetzt ist. Hierdurch wird eine Drehbewegung des Kegelantriebsrads über das Ringkegelrad auf den Aussenzahnkranz übertragen und das Schneidrohr 5 um seine Längsachse gedreht.

Des weiteren ist erkennbar, dass das Schneidrohr 5 über eine Rastverbindung lösbar in das Handmodul 1 bzw. das Aufnahmeelement einsetzbar ist. Hierzu stehen von dem Schneidrohr 5 eine Rastfeder 16 ab, die in Zusammenbaulage die nach innen als Rastnocke vorstehende Rastnut hintergreifen.

Nach dem Einsetzen des Schneidrohrs 5 in beispielsweise einen menschlichen Körper zum Entnehmen von Gewebe, beispielsweise durch einen Trokar, wird über einen Druckknopf der Antrieb bzw. dessen Drehachse in Drehbewegung versetzt. Dabei dreht auch das Kegelantriebsrad und treibt über das Ringkegelrad, dessen Innenzahnkranz und den Aussenzahnkranz das Schneidrohr 5 an bzw. versetzt dieses in Drehbewegung.
Sollte es notwendig sein, kann durch ein entsprechendes Ventil in dem Ventilmodul 4 ein weiteres chirurgisches Instrument eingesetzt werden, welches dann auch durch das Schneidrohr 5 bis zu dessen distalem Ende hindurch geführt werden kann. Des weiteren befindet sich am distalen Ende des Schneidrohrs 5 eine Schneide. Dieses Schneide ist aus Federstahl gebildet und zu einer Hülse zusammengerollt.
Ansonsten wird bzgl. der Einzelheiten auf die Offenbarung der DE 10 2010 037 974 A1 verwiesen. Dort ist ein Morcellator beschrieben.
In Figur 6 ist eine Schutzhülse S mit einem darin befindlichen medizinischen Instrument I in Gebrauchslage dargestellt. Die Schutzhülse S umfasst einen Tubus 101 sowie proximal-seitig eine Verbindungseinrichtung 102.

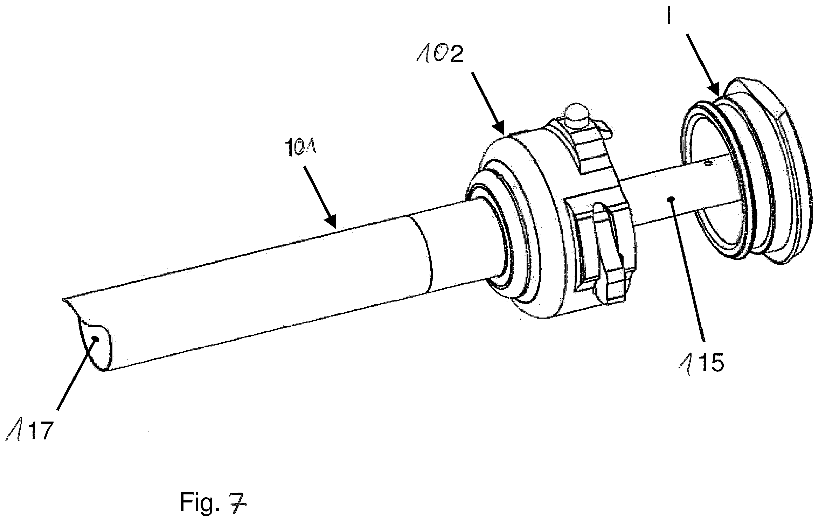

In Figur 7 ist eine Schutzhülse S und ein medizinisches Instrument I nach Figur 6 in Ausgangslage gezeigt. Weiter ist in der Figur 7 ein Schaft 115 und ein Hohlraum 117 gezeigt.

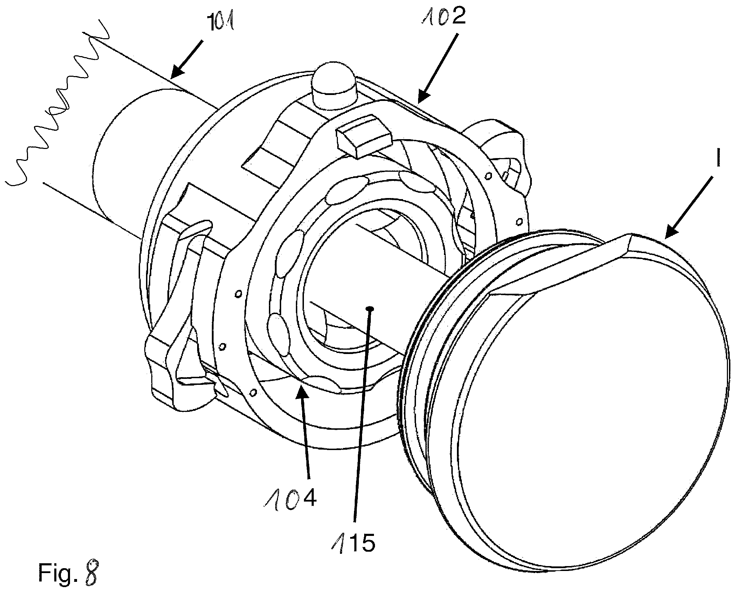

Figur 8 zeigt eine perspektivische Teilansicht des proximalen Endes der Schutzhülse S nach Figur 6 in Ausgangslage. Die Verbindungseinrichtung 102 und ein von ihr im Wesentlichen umfangene Ventil 104 sind deutlich zu erkennen.

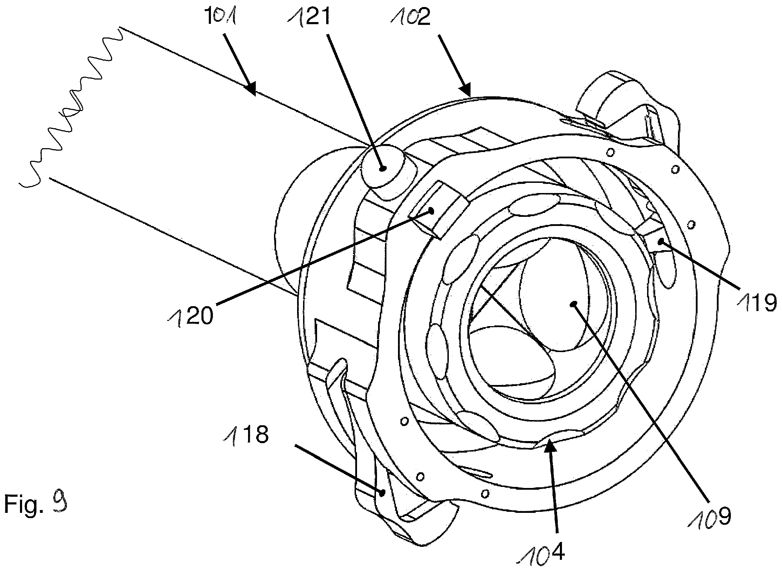

In Figur 9 ist eine Ansicht nach Figur 8 in Ausgangslage ohne medizinisches Instrument I dargestellt. Eine Dichteinheit 109 des Ventils 104 ist erkennbar. Der Verbindungseinrichtung 102 ist ein Nocken 120 angeformt. Ferner sind ihr Wipprasten 118 mit Einrastern 119 zugeordnet.

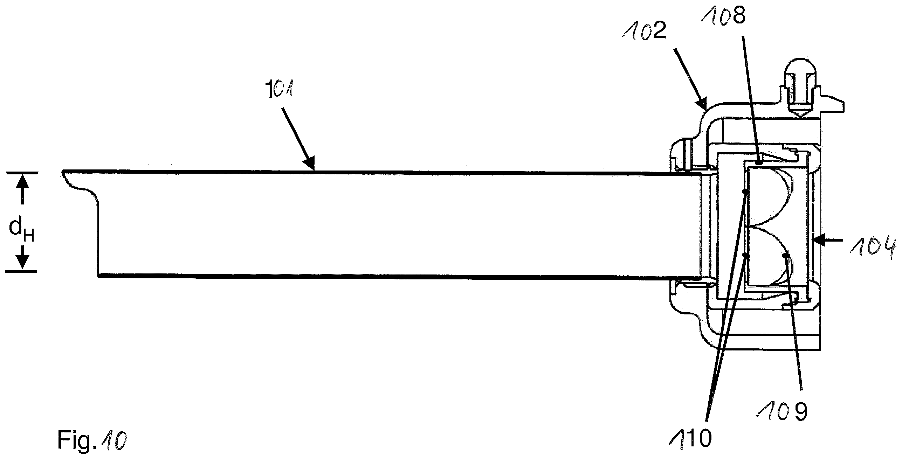

Figur 10 zeigt eine geschnittene Seitenansicht einer Schutzhülse S in Ausgangslage. Im Inneren einer Aufnahme 108 des Ventils 104 befindet sich die Dichteinheit 109 mit den Dichtlippen 110. Weiter ist ein Durchmesser des Hohlraum dH gezeigt.

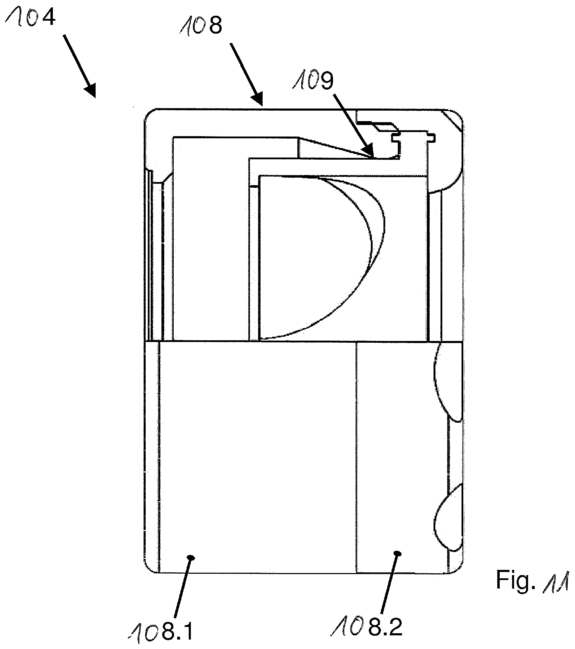

Figur 11 zeigt eine teilweise geschnittene Seitenansicht eines Ventils 104 in Ausgangslage. Ein Oberteil 108.2 und ein Unterteil 108.1 bilden gemeinsam die Aufnahme 108. Ausserdem ist eine Dichteinheit 109 gezeigt.

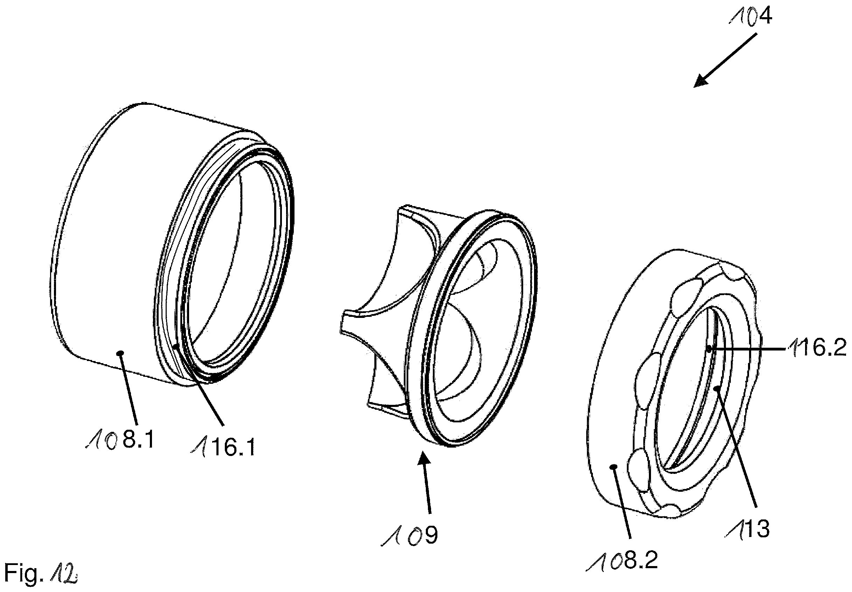

In Figur 12 ist eine perspektivische Ansicht eines Ventils 104 im auseinander gebauten Zustand dargestellt. Dem Unterteil 108.1 ist ein Aussengewinde 116.1 angeformt. Dem Oberteil 108.2 ist seinerseits ein Innengewinde 116.2 angeformt. Weiter ist die Dichteinheit 109 gezeigt, welche zwischen dem Unterteil 108.1 und dem Oberteil 108.2 angeordnet ist. Wenn das Unterteil 108.1 und das Oberteil 108.2 miteinander verschraubt werden, wird die Dichteinheit 109 zwischen dem Unterteil 108.1 und dem Oberteil 108.2 festgelegt.

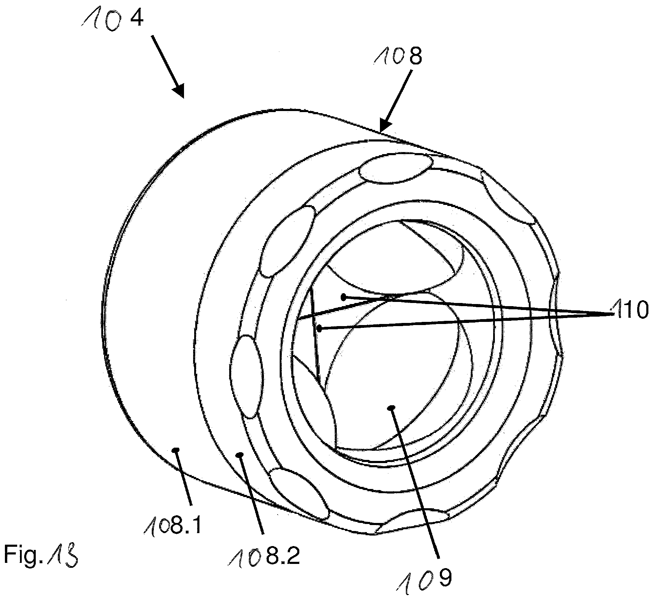

Figur 13 zeigt eine perspektivische Ansicht eines Ventils 104 im zusammengebauten Zustand mit sich berührenden Dichtlippen 110, wenn das Unterteil 108.1 und das Oberteil 108.2 miteinanderverschraubt werden und die Dichteinheit 109 zwischen sich festlegen.

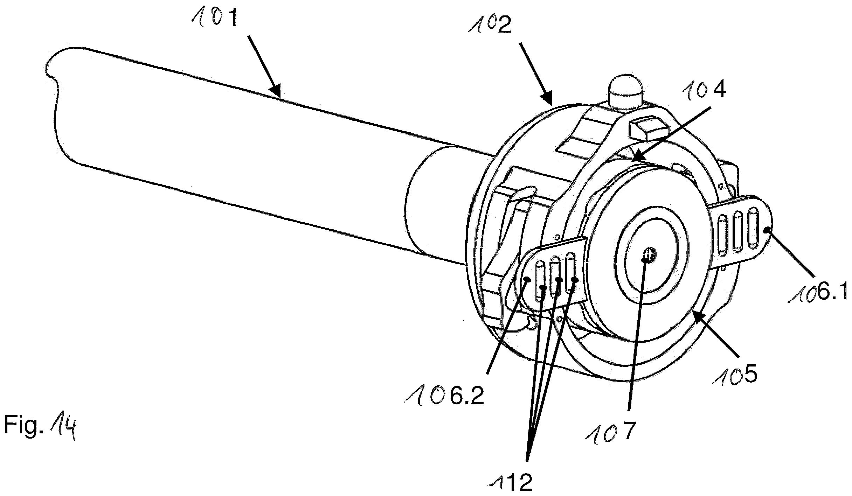

Figur 14 zeigt eine perspektivische Ansicht einer Schutzhülse S mit Ventil 104 und Abdichtelement 105 in einer ersten Ausgangslage. Das Abdichtelement 105 ist mit dem Ventil 104 verbunden. Seitlich sind dem Abdichtelement 105 Laschen 106.1, 106.2 angeformt, welche Erhebungen 112 aufweisen. Mittig verfügt das Abdichtelement 105 über eine Ausnehmung 107.

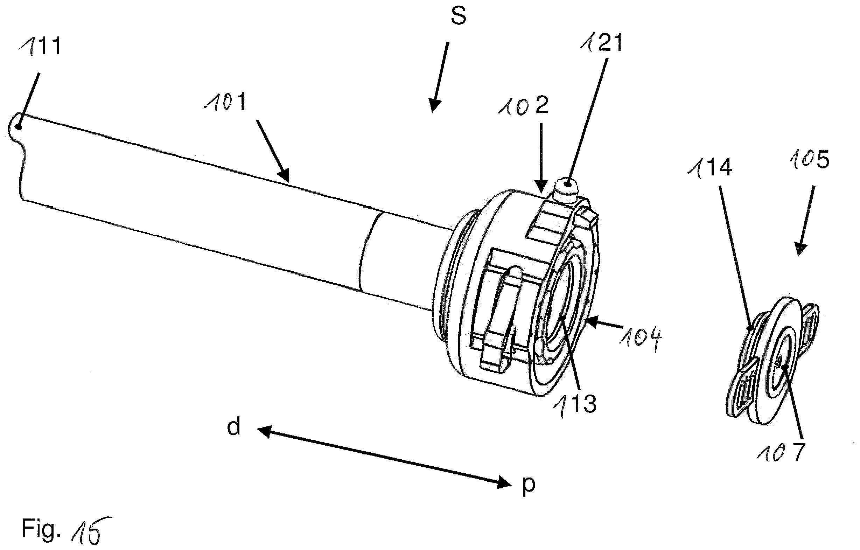

Figur 15 eine perspektivische Ansicht einer Schutzhülse S mit Ventil 104 und Abdichtelement 105 in einer zweiten Ausgangslage. Eine Nut 113 des Ventils 104 und ein Wulst 114 des Abdichtelements 105 sind deutlich zu erkennen.

Bezugnehmend auf die Figuren 6 - 15 erklärt sich die Funktionsweise der erfindungsgemässen Vorrichtung folgendermassen:
Die Schutzhülse S dient einer Aufnahme eines nicht näher gezeigten medizinischen bzw. chirurgischen Instruments I. Der Tubus 101 stellt im Rahmen einer minimal-invasiven Operation die Verbindung zwischen einem Ort der Operation, an welchem beispielsweise Gewebe entnommen wird, also beispielsweise einer Körperhöhle eines Patienten, und einem Operationssaal bzw. einer Umgebung des Patienten her. Der Tubus 101 umfängt hierbei ein medizinisches Instrument I und schirmt zugleich intaktes Gewebe des Patienten gegenüber dem medizinischen Instrument I ab.

Ist, wie in den Figuren 9 und 10 erkennbar, kein medizinisches Instrument I in die Schutzhülse S eingebracht, so schliessen die Dichtlippen 110 der Dichteinheit 109 des Ventils 104. Ein Gasdurchtritt eines in der Körperhöhle befindlichen Gases, vom distalen zum proximalen Ende der Schutzhülse S wird somit verhindert.

Zwecks Austausch oder Reinigung können die mit korrespondierenden Gewinden 116.1, 116.2 versehenen Ober- 108.2 bzw. Unterteile 108.1 des Ventils 104 getrennt werden. Die Dichteinheit 109 kann gereinigt oder ausgetauscht werden.

Das Abdichtelement 105 kann bei Bedarf auf das Ventil 104 aufgesetzt werden. Eine Verbindung beider Teile wird durch ein Zusammenwirken eines Wulstes 114 des Abdichtelements mit einer Nut 113 des Ventils 104 bewerkstelligt. Um Abdichtelement 105 und Ventil 104 zu konnektieren bzw. zu verbinden, wird das aus einem flexiblem Werkstoff bestehende Abdichtelement 105 in distaler Richtung in ein proximales Ende des Ventils 104 eingebracht. Die Ausnehmung 107 des Abdichtelements 105 ermöglicht ein Führen eines medizinischen oder beispielsweise optischen Instruments mit einem zum Querschnitt des Hohlraums 117 des Tubus 101 sehr kleinem Querschnitt. Ein Instrument wird somit im Wesentlichen koaxial mit einer gedachten Längsachse des Tubus 101 geführt. Ferner ermöglicht das Abdichtelement 105 eine zusätzliche Abdichtung zweier jenseits von distalem und proximalem ende der Schutzhülse S befindlichen Räume, beispielsweise Operationssaal und Körperhöhle, indem ein Schaftdurchmesser des Instruments und ein Durchmesser der Ausnehmung 107 des Abdichtelements 105 derart gewählt sind, dass ein in die Ausnehmung 107 eingebrachtes Instrument einen Durchtritt von Gas durch die Ausnehmung 107 verhindern kann.

Mittels der Laschen 106.1, 106.2 kann ein Operateur die formschlüssige Verbindung zwischen Abdichtelement 105 und Ventil 104 auch während einer Operation leicht wieder lösen. Hierbei sind die Erhebungen 112 auf den Laschen 106.1, 106.2 hilfreich, da sie ein Abrutschen von Fingern des Operateurs verhindern bzw. sicheren Halt geben.

Um die Schutzhülse S mit einem nicht dargestellten Handstück zu verbinden, kann der Nocken 120 in eine nicht dargestellte Ausnehmung des Handstücks eingebracht werden. Dies verhindert ein unerwünschtes Drehen der Schutzhülse S gegenüber dem Handstück während einer Operation. Die Verbindung zwischen Handstück und Schutzhülse S wird durch Wipprasten 118 geschaffen, denen einends Einraster 119 angeformt sind. Die Einraster 119 können in eine nicht gezeigte Nut des Handstücks eingreifen und die Schutzhülse S mit dem Handstück verbinden. Ein Positionsindikator 121 ist zweckdienlich, wenn beispielsweise eine Abdeckung 111 vorhanden ist. Befinden sich Positionsindikator 121 und Abdeckung 111 in einem gedachten Längsschnitt durch die Schutzhülse S in einer Ebene, so ist mittels des während einer Operation ausserhalb der Körperhöhle befindlichen Positionsindikators 121 für den Operateur stets erkennbar, wo sich die Abdeckung 111 befindet.

**Positionszahlenliste**

| | | | | | |
|---|---|---|---|---|---|
| 1 | Handmodul | 101 | Tubus | | |
| 2 | Griffteil | 102 | Verbindungseinrichtung | | |
| 3 | Führungsteil | 103 | Schleuseneinheit | | |
| 4 | Ventilmodul | 104 | Ventil | | |
| 5 | Schneidrohr | 105 | Abd ichtelement | | |
| 6 | Antriebsanschlusses | 106 | Lasche | | |
| 7 | Schneide | 107 | Ausnehmung | | |
| 8 | Hülse | 108 | Aufnahme | | |
| 9 | Konnektionsring | 109 | Dichteinheit | | |
| 10 | Konektionsstück | 110 | Dichtlippen | | |
| 11 | Anflanschring | 111 | Abdeckung | | |
| 12 | Ausnehmung | 112 | Erhebung | | |
| 13 | Flanschnocken | 113 | Nut | | |
| 14 | Betätigungsknopf | 114 | Wulst | | |
| 15 | Wippraste | 115 | Schaft | | |
| 16 | Rastnocken | 116 | Gewinde | | |
| 17 | Abdeckung | 117 | Hohlraum | | |
| 18 | Wulst | 118 | Wippraste | | |
| 19 | | 119 | Einraster | | |
| 20 | Nut | 120 | Nocken | | |
| 21 | Einraster | 121 | Positionsindikator | | |
| 22 | | 122 | | | |
| 23 | | 123 | | | |
| 24 | | 124 | | M | Morcellator |
| 25 | | 125 | | | |
| 26 | | 126 | | S | Schutzhülse |
| 27 | | 127 | | I | Instrument |
| 28 | | 128 | | p | proximal |
| 29 | | 129 | | d | distal |
| 30 | | 130 | | d_{H} | Durchmesser Hohlraum |
| 31 | | 131 | | | |
| 32 | | 132 | | | |
| 33 | | 133 | | | |

## Patentansprüche

1. Morcellator (M) mit einem Konnektionselement, wobei der Morcellator (M) ein Handmodul (1), ein Schneidrohr (5) und eine Hülse (8) aufweist,
**dadurch gekennzeichnet,**
**dass** das Handmodul (1) ein Konnektionsstück (10) umfasst und die Hülse (8) einen Konnektionsring (9) umfasst, wobei der Konnektionsring (9) auf das Konnektionsstück (10) aufclipsbar ist, wobei das Konnektionsstück (10) einen Anflanschring (11) mit Ausnehmungen (12) aufweist, wobei in die Ausnehmungen (12) ein Flanschnocken (13) des Konnektionsrings (9) durch das Aufclipsen des Konnektionsrings (9) auf das Konnektionsstück (10) drehfest in Eingriff bringbar ist

2. Konnektionselement nach Anspruch 1, **dadurch gekennzeichnet, dass** der Flanschnocken (13) in der Verlängerung des Umfangs der Hülse (8) eine Abdeckung (17) aufweist, wobei der Flanschnocken (13) und die Abdeckung (17) im Wesentlichen eine Linie bilden.

3. Konnektionselement nach Anspruch 2, **dadurch gekennzeichnet, dass** die Abdeckung (17) distalseitig eine teilweise Verlängerung der Hülse (8) auf einem Teil des Umfangs der Hülse (8) darstellt.

4. Konnektionselement nach einem der Ansprüche 1 bis 3, **dadurch gekennzeichnet, dass** der Flanschnocken (13) eine Positionsanzeige in Form einer Wulst (18) aufweist, wobei die Wulst (18) die Position des Flanschnockens (13) und der Abdeckung (17) anzeigt.

5. Konnektionselement nach einem der vorigen Ansprüche, **dadurch gekennzeichnet, dass** das Schneidrohr (5) über einen Rastnocken (16) mit dem Handmodul (1) verclipsbar ist.

6. Konnektionselement nach einem der vorigen Ansprüche, **dadurch gekennzeichnet, dass** das Konnektionsstück (10) über einen Betätigungsknopf (14) von dem Handmodul (1) weg oder zu dem Handmodul (1) hin setzbar ist.

7. Konnektionselement nach Anspruch 6, **dadurch gekennzeichnet, dass** der Betätigungsknopf (14) eine Schraubbewegung des Konnektionsstücks (10) an dem Handmodul (1) bewirkt und somit eine Beabstandung erreicht.

8. Konnektionselement nach einem der Ansprüche 6 oder 7, **dadurch gekennzeichnet, dass** der Betätigungsknopf (14) durch das Wegsetzen des Konnektionsstücks (10) von dem Handmodul (1) auch den Konnektionsring (9) von dem Handmodul (1) wegsetzt, wobei die Hülse (8) über eine Schneide (7) des Schneidrohrs (5) schiebbar ist.

9. Konnektionselement nach einem der Ansprüche 6 bis 8, **dadurch gekennzeichnet, dass** der Betätigungsknopf (14) durch das Hinsetzen des Konnektionsstücks (10) zu dem Handmodul (1) auch den Konnektionsring (9) zu dem Handmodul (1) hinsetzt, wobei die Hülse (8) eine Schneide (7) des Schneidrohrs (5) freigibt.

10. Konnektionselement nach einem der vorigen Ansprüche, **dadurch gekennzeichnet, dass** der Konnektionsring (9) eine Wippraste (15) aufweist, welche zur Festlegung des Konnektionsrings (9) an dem Konnektionsstück (10) dient, wobei die Wippraste (15) in eine Nut (20) des Konnektionsstücks (10) eingreift.

## Claims

1. Morcellator (M) having a connecting element, wherein the morcellator (M) comprises a hand module (1), a cutting tube (5) and a sleeve (8),
**characterised**
**in that** the hand module (1) comprises a connecting piece (10) and the sleeve (8) comprises a connecting ring (9), wherein the connecting ring (9) can be clipped onto the connecting piece (10), wherein the connecting piece (10) has a flange-mounted ring (11) having recesses (12), wherein a flange cam (13) of the connecting ring (9) can be brought into engagement in a manner fixed against rotation in the recesses (12) by clipping the connecting ring (9) onto the connecting piece (10).

2. Connecting element according to claim 1, **characterised in that** the flange cam (13) has a covering (17) in the extension of the circumference of the sleeve (8), wherein the flange cam (13) and the covering (17) substantially form a line.

3. Connecting element according to claim 2, **characterised in that** the covering (17) has at a distal end a partial extension of the sleeve (8) over part of the circumference of the sleeve (8).

4. Connecting element according to one of claims 1 to 3, **characterised in that** the flange cam (13) has a position indicator in the form of a bead (18), wherein the bead (18) indicates the position of the flange cam (13) and of the covering (17).

5. Connecting element according to one of the preceding claims, **characterised in that** the cutting tube (5) can be clipped to the hand module (1) via a latching cam (16).

6. Connecting element according to one of the preceding claims, **characterised in that** the connecting piece (10) is movable away from the hand module (1) or towards the hand module (1) via an actuating button (14).

7. Connecting element according to claim 6, **characterised in that** the actuating button (14) brings about a screwing movement of the connecting piece (10) on the hand module (1) and therefore achieves a spacing.

8. Connecting element according to one of claims 6 or 7, **characterised in that** the actuating button (14), by moving the connecting piece (10) away from the hand module (1), also moves the connecting ring (9) away from the hand module (1), wherein the sleeve (8) can be pushed over a cutting edge (7) of the cutting tube (5).

9. Connecting element according to one of claims 6 to 8, **characterised in that** the actuating button (14), by moving the connecting piece (10) towards the hand module (1), also moves the connecting ring (9) towards the hand module (1), wherein the sleeve (8) releases a cutting edge (7) of the cutting tube (5).

10. Connecting element according to one of the preceding claims, **characterised in that** the connecting ring (9) has a rocker latch (15) which serves for securing the connecting ring (9) on the connecting piece (10), wherein the rocker latch (15) engages in a groove (20) of the connecting piece (10).

## Revendications

1. Morcellateur (M) avec un élément de connexion, dans lequel le morcellateur (M) présente un module à main (1), un tuyau de coupe (5) et un manchon (8),
**caractérisé par le fait**
**que** le module manuel (1) comporte une pièce de connexion (10) et que le manchon (8) comporte une bague de connexion (9), la bague de connexion (9) pouvant être clipsée sur la pièce de connexion (10), la pièce de connexion (10) présentant une bague de bridage (11) avec des évidements (12), dans les évidements (12) pouvant être engagée de manière fixe en rotation une came de bride (13) de la bague de connexion (9) par le clipsage de la bague de connexion (9) sur la pièce de connexion (10).

2. Elément de connexion selon la revendication 1, **caractérisé par le fait que** la came de bride (13) présente, dans le prolongement de la circonférence du manchon (8), un recouvrement (17), la came de bride (13) et le recouvrement (17) formant sensiblement une ligne.

3. Elément de connexion selon la revendication 2, **caractérisé par le fait que** le recouvrement (17) représente, du côté distal, un prolongement partiel du manchon (8) sur une partie de la circonférence du manchon (8).

4. Elément de connexion selon l'une des revendications 1 à 3, **caractérisé par le fait que** la came de bride (13) présente un indicateur de position sous forme d'un bourrelet (18), le bourrelet (18) indiquant la position de la came de bride (13) et du recouvrement (17).

5. Elément de connexion selon l'une des revendications précédentes, **caractérisé par le fait que** le tuyau de coupe (5) peut être clipsé par l'intermédiaire d'une came d'encliquetage (16) au module à main (1).

6. Elément de connexion selon l'une des revendications précédentes, **caractérisé par le fait que** la pièce de connexion (10) peut être placée par l'intermédiaire d'un bouton d'actionnement (14) éloignée du module à main (1) ou vers le module à main (1).

7. Elément de connexion selon la revendication 6, **caractérisé par le fait que** le bouton d'actionnement (14) provoque un mouvement de vissage de la pièce de connexion (10) sur le module à main (1) et atteint ainsi un écartement.

8. Elément de connexion selon l'une des revendications 6 ou 7, **caractérisé par le fait que** le bouton d'actionnement (14) écarte, par l'écartement de la pièce de connexion (10) du module à main (1), également la bague de connexion (9) du module à main (1), le manchon (8) pouvant être glissé sur une arête de coupe (7) du tuyau de coupe (5).

9. Elément de connexion selon l'une des revendications 6 à 8, **caractérisé par le fait que** le bouton d'actionnement (14) approche, par l'approche de la pièce de connexion (10) du module à main (1), également la bague de connexion (9) du module à main (1), le manchon (8) libérant une arête de coupe (7) du tuyau de coupe (5).

10. Elément de connexion selon l'une des revendications précédentes, **caractérisé par le fait que** la bague de connexion (9) présente un cran basculant (15) qui sert à fixer la bague de connexion (9) sur la pièce de connexion (10), ledit cran basculant (15) s'engageant dans une rainure (20) de la pièce de connexion (10).
